# EUROPEAN PATENT APPLICATION

(11) **EP 0 784 994 A1**
(43) Date of publication of application: **23.07.1997**
(21) Application number: 96850217.9
(22) Date of filing: 20.12.1996
(51) Int. Cl.: A61N 1/05

(54) **Electrode cable**

(30) Priority: 28.12.1995 SE 9504678
(71) Applicant: Pacesetter AB, 171 95 Solna (SE)
(72) Inventor: Lindegren, Ulf, 121 33 Enskededalen (SE)
(74) Representative: Winblad, Hans Peter

(57) **Abstract**

The invention relates to an electrode cable (10), intended for implantation in a body cavity. The cable contains at least one elongate electrical conductor (12, 14, 16, 18) and a means (22) for temporarily stiffening the conductor during introduction of same into the body cavity. According to the invention, the means (22) for temporarily stiffening the conductor consists of a biodegradable, biocompatible polymer material attached to the exterior of the conductor.

## Description

### TECHNICAL FIELD

The present invention relates to an electrode cable, intended for implantation in a body cavity, specially for intracardiac stimulation and/or sensing of heart signals, said cable containing at least one elongate, flexible electrical conductor with a distal end and a proximal end, an electrode being arranged at the distal end of the conductor for fixation to tissue in a heart wall, and a means for temporarily stiffening the conductor at the insertion of same into the body cavity.

### THE PRIOR ART

Electrode cables of the kind in common use (see e.g. US-A-4 136 703) are normally composed of one or more coaxially, helically coiled electrical conductors which are externally enclosed in a tubular silicone rubber sleeve and internally form a hollow, central channel. These electrode cables are therefore soft and flexible. In order to facilitate introduction of this electrode cable into e.g. the heart via veins and reach the desired site for the distal end electrode against the myocardial wall, a stylet, introduced into the electrode cable's central channel, is used, thereby stiffening the cable to the requisite degree during the implantation process. The stylet is then withdrawn. However, superfluous channel space remains.

### SUMMARY OF THE INVENTION

One object of the present invention is to achieve an electrode cable which, in comparison to known electrode cables with the same number of conductors, is thinner and more flexible than those and which does not need a stylet to achieve temporary stiffening during the electrode cable's introduction and implantation.

For this purpose, the aforementioned electrode cable according to the invention is distinguished in that the means for temporarily stiffening the conductor consists of a biodegradable, biocompatible polymer material, attached to the exterior of the conductor, the polymer material preferably in the shape of a tubular sleeve which at least partially encloses the conductor. When such a material with an appropriate degradation time, e.g. one or several hours, is selected, a much slimmer and more flexible electrode cable is achieved, after the tubular sleeve had been degraded or dissolved, than previously, and the conductor or conductors can consist of individual straight or twisted wire means which fit in the spaces which would otherwise have served as a central stylus channel.

Examples of biodegradable, biocompatible polymer materials which could be used in an application according to the present invention are stated in dependent patent claims 3-7 below.

### FIGURES

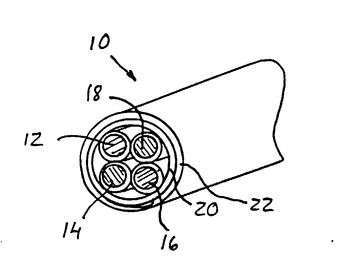

The attached drawing is a schematic, perspective view, on an enlarged scale, of a small part of an electrode cable devised according to the present invention.

### PREFERRED EMBODIMENT

The drawing shows an electrode cable 10, according to the present invention, which here has four insulated, straight or intertwined electrical conductors 12, 14, 16, 18 enclosed in an tubular sleeve 20 made of e.g. silicone rubber.

A tube 22, threaded or molded onto the exterior of the sleeve 20 according to the invention, made of a biodegradable, biocompatible polymer material which makes the electrode cable 10 appropriately stiff during implantation but which, after about one hour or more when its stiffening function is superfluous, dissolves from contact with blood. The electrode cable 10 accordingly acquires a slimmer and more flexible construction. The fact that no conventional stylet is required, i.e. the space corresponding to the normal, central stylet channel formed by helically coiled conductors can instead be used to house the conductors, straight or twisted, in the axial direction, also contributes to this. Alternately, devising the biodegradable polymer material as bands or strips, which extend along the exterior of the sleeve 20 in any configuration appropriate to the objective and lend the electrode cable the desired stiffness during implantation, is also conceivable within the scope of the invention.

The biodegradable polymer material can be selected from e.g. the groups proteins/amino acid polymers, poly(hydroxycarboxyl acids) and/or carbohydrate polymers. The proteins/amino acid polymers group can contain gelatin, collagen, polyserine, polythreonine, polyphenylalanine or the like. Moreover, the poly(hydroxycarboxyl acids) group may contain polylactides and/or polyglycolides. The carbohydrate polymers group can contain dextran, starch, hyaluronic acid, cellulose or the like. The breakdown or degradation time for the polymer material in the sleeve 22 should be relatively short but exceed about one hour.

Electrode cables of the aforementioned kind are especially suitable for implantation in the heart ventricle.

## Claims

1. An electrode cable, intended for implantation in a body cavity, especially for intracardiac stimulation and/or sensing of heart signals, containing at least one elongate, flexible electrical conductor (12, 14, 16, 18) with a distal end and a proximal end, an electrode being arranged on the distal end of the conductor for fixation to tissue in a heart wall, and a means for temporarily stiffening the conductor (12, 14, 16, 18) during the introduction of same into the body cavity, **characterized** in that the means (22) for temporarily stiffening the conductor (12, 14, 16, 18) consists of a biodegradable, biocompatible polymer material attached to the exterior of the conductor.

2. An electrode cable according to claim 1, **characterized** in that the polymer material has the shape of a tubular sleeve enclosing the conductor (12, 14, 16, 18) at least in part.

3. An electrode cable according to claim 1, **characterized** in that the polymer material is selected from the groups proteins/amino acid polymers, poly(hydroxycarboxyl acids) and/or carbohydrate polymers.

4. An electrode cable according to claim 3, **characterized** in that the proteins/amino acid polymers group contains gelatin, collagen, polyserine, polythreonine, polyphenylalanine or the like.

5. An electrode cable according to claim 3, **characterized** in that the poly(hydroxycarboxyl acids) group contains polylactides and/or polyglycolides.

6. An electrode cable according to claim 3, **characterized** in that the carbohydrate polymers group contains dextran, starch, hyaluronic acid, cellulose or the like.

7. An electrode cable according to any of claims 1-6, **characterized** in that the polymer material in the tubular means (22) has a degradation time greater than at least one hour.
